(19) **Europäisches Patentamt**

**European Patent Office**

**Office européen des brevets**

(11) **EP 1 073 438 B1**

(12) **EUROPEAN PATENT SPECIFICATION**

(45) Date of publication and mention
of the grant of the patent:
**31.03.2004 Bulletin 2004/14**

(51) Int Cl.⁷: **A61K 31/445**, A61K 9/20

(21) Application number: **99933484.0**

(86) International application number:
**PCT/US1999/012368**

(22) Date of filing: **08.07.1999**

(87) International publication number:
**WO 2000/002560 (20.01.2000 Gazette 2000/03)**

(54) **8-CHLORO-6,11-DIHYDRO-11-(4-PIPERIDYLIDINE)-5H-BENZO (5,6) CYCLOHEPTA (1,2-b) PYRIDINE ORAL COMPOSITIONS**

ORALE ZUSAMMENSETZUNGEN MIT
8-CHLORO-6,11-DIHYDRO-11-(4-PIPERIDYLIDEN)-5H-BENZO[5,6]CYCLOHEPTA[1,2-B]PYRIDIN

COMPOSITIONS ORALES DE 8-CHLORO-6,11-DIHYDRO-11-(4-PIPERIDYLIDENE)-5H-BENZO 5,6]CYCLOHEPTA 1,2-b]PIRYDINE

(84) Designated Contracting States:
**AT BE CH CY DE DK ES FI FR GB GR IE IT LI LU MC NL PT SE**
Designated Extension States:
**LT LV RO**

(30) Priority: **10.07.1998 US 113232**

(43) Date of publication of application:
**07.02.2001 Bulletin 2001/06**

(60) Divisional application:
**03023240.9 / 1 380 297**

(73) Proprietor: **SCHERING CORPORATION**
**Kenilworth, New Jersey 07033-0530 (US)**

(72) Inventor: **KOU, Jim, H.**
**Basking Ridge, NJ 07920 (US)**

(74) Representative: **Ritter, Stephen David et al**
**Mathys & Squire**
**100 Gray's Inn Road**
**London WC1X 8AL (GB)**

(56) References cited:
**EP-A- 0 396 404          WO-A-98/34614**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**EP 1 073 438 B1**

**Description**

This invention relates to pharmaceutical compositions containing 8-chloro-6,11-dihydro-11-(4-piperidylidene)-5H-benzo[5,6]-cyclohepta[1,2-b]pyridine (hereinafter "descarbonylethoxyloratadine" or ("DCL") and substantially free of DCL decomposition products, and suitable for oral administration to treat allergic reactions in mammals.

U.S. Patent No. 4,659,716 discloses descarbonylethoxy-loratadine which possesses antihistaminic properties with substantially no sedative properties. This U.S. Patent also discloses methods of making descarbonyl-ethoxyloratadine, pharmaceutical compositions it and methods of using the compositions to treat allergic reactions in mammals.

U.S. Patent No 5,595,997 discloses pharmaceutical compositions and methods for treating allergic rhinitis using descarbonylethoxyloratadine. Co-pending, commonly-owned U.S. Patent application Serial No. 08/886,766, filed 07/02/97 discloses polymorphs of descarbonyl-ethoxyloratadine and pharmaceutical compositions containing them.

EP 0 396 404 discloses pharmaceutical compositions comprising loratadine, or the decarbalkoxylation product thereof, in combination with ibuprofen and pseudoephedrine for the treatment of cough and cold symptoms.

WO 98/34614, which was published after the present priority date, discloses stable pharmaceutical compositions of descarboethoxyloratadine (DCL), which are formulated to avoid the incompatibility between DCL and reactive excipients such as lactose and other mono- and di-saccharides. The compositions may be lactose-free, non-hygroscopic and anhydrous.

We are aware of no prior art that discloses the pharmaceutical compositions of the present invention.

There is a need to produce pharmaceutical compositions suitable for oral administration to mammals and containing descarbonylethoxyloratadine having constant chemical and physical properties in accordance with exacting health registration requirements of the U.S. and international health registration authorities, e.g. , the FDA's Good Manufacturing Practices ("GMP") requirements and the International Conference on Harmonization ("ICH") Guidelines.

**Summary of the Invention**

We have found that descarbonylethoxyloratadine discolors and decomposes in the presence of excipients disclosed in the prior art. We have discovered that these problems are substantially solved when the use of an acidic excipient is avoided and descarbonylethoxyloratadine is combined with a pharmaceutically acceptable carrier medium comprising a DCL-protective amount of a pharmaceutically acceptable basic salt. Thus, this invention provides a pharmaceutical composition comprising an anti-allergic effective amount of descarbonylethoxyloratadine in a pharmaceutically acceptable carrier medium comprising a pharmaceutically acceptable basic salt,
wherein the pharmaceutically acceptable carrier medium is substantially free of acidic excipients, and wherein the pharmaceutically acceptable basic salt is a carbonate, phosphate, silicate or sulfate of calcium, magnesium or aluminum or mixtures thereof.

The pharmaceutical compositions of the present invention contain less than about 1 % of decomposition products such as N-formylDCL initially, as well as when such compositions are stored at 25°C and about 60 % relative humidity for period of at least 24 months.

In a preferred embodiment, the pharmaceutical compositions of the present invention comprise an anti-allergic effective amount of descarbonylethoxy-loratadine in a pharmaceutically acceptable carrier medium wherein said composition contains less than about 1% by weight of N-formyl DCL, preferably less than about 0.8% of N-formyl DCL, and more preferably less than about 0.6% of N-formyl DCL.

In another preferred embodiment, the pharmaceutical compositions of the present invention are adapted for oral administration and which further comprise at least one disintegrant. The at least one pharmaceutically acceptable disintegrant is added in an amount sufficient to provide dissolution of at least about 80% by weight of the pharmaceutical composition in about 45 minutes.

This invention also provides a pharmaceutical composition for oral administration as defined above comprising an anti-allergic effective amount of descarbonylethoxyloratadine in a pharmaceutically acceptable carrier medium comprising a DCL- protective amount of a calcium dibasic phosphate, and an amount of microcrystalline cellulose and of starch sufficient to provide dissolution of at least about 80% by weight of the pharmaceutical composition in about 45 minutes.

In a preferred embodiment, this invention provides a pharmaceutical composition as defined above for oral administration comprising an anti-allergic effective amount of descarbonylethoxyloratadine in a pharmaceutically acceptable carrier medium comprising a DCL- protective amount of a calcium dibasic phosphate, and an amount of microcrystalline cellulose and of starch sufficient to provide dissolution of at least about 80% by weight of the pharmaceutical composition in about 45 minutes, and which contains less than about 1% by weight of N-formyldescarbonyl-ethoxyloratadine.

This invention further provides a preferred pharmaceutical composition for oral administration comprising:

| Ingredient | Amount (weight %) |
|---|---|
| Descarbonylethoxyloratadine | about 0.5 - 15 |
| Calcium Dibasic Phosphate Dihydrate USP | about 10 - 90 |
| Microcrystalline Cellulose NF | about 5 - 60 |
| Corn starch NF | about 1 - 60 |
| Talc USP | about 0.5 - 20 |

This invention also provides another preferred pharmaceutical composition for oral administration comprising:

| Ingredient | Amount (weight %) |
|---|---|
| Descarbonylethoxyloratadine | about 0.5 -15 |
| Calcium Dibasic Phosphate Dihydrate USP | about 45 - 60 |
| Microcrystalline Cellulose NF | about 20 - 40 |
| Corn starch NF | about 5 - 15 |
| Talc USP | about 1 - 10 |

This invention also provides another preferred pharmaceutical composition for oral administration comprising:

| Ingredient | Amount (weight %) |
|---|---|
| Descarbonylethoxyloratadine | about 1 - 10 |
| Calcium Dibasic Phosphate Dihydrate USP | about 50 - 56 |
| Microcrystalline Cellulose NF | about 25 - 35 |
| Corn Starch NF | about 10 - 12 |
| Talc USP | about 2 - 5 |

The pharmaceutical compositions of the present invention are useful for treating allergic reactions in mammals.

## Detailed Description of the Invention

During the development of the compositions of the present invention, descarbonylethoxyloratadine was found to discolor when stored at 75% relative humidity ("RH") and a temperature of 40°C, alone or in combination with various excipients, such as those disclosed in U.S. Patent Nos. 4,657,716 and 5,595,997. We discovered that this color instability in the active ingredient was apparently due to a very minute amount of a degradation product caused by the presence of a wide variety of excipients commonly used in oral formulations - especially a tablet formulation. These excipients found unsuitable include acidic excipients including, but not limited to, stearic acid, povidone, and crospovidone, and other acidic excipients having a pH in water fewer than 7, and preferably in the range of about 3 to 5 as well as other excipients such as lactose, lactose monohydrate, sodium benzoate, and Glyceryl Behenate NF sold under the tradename of Compritol 888 .The presence of acidic excipients such as stearic acid in a solid powder formulation blend (similar to that of Example 6) containing DCL, lactose monohydrate, and stearic acid resulted in a large amount (14%) of decomposition of descarbonylethoxyloratadine after one week at 40°C and 75 % RH. When the pharmaceutical compositions of the present invention were subjected to the same stressed conditions for a longer period of time, i.e., 3 months, less than about 1% decomposition of descarbonylethoxyloratadine was found in the pharmaceutical compositions of the present invention. See Examples 1-5, 6 and 10 hereinafter. The pharmaceutically acceptable carrier medium used in the pharmaceutical compositions of the present invention should be substantially free, i.e., contain less than about 1 % by weight, of acidic excipients.

The major decomposition product of DCL found in the pharmaceutical compositions of the present invention is N-formylDCL. The pharmaceutical compositions of the present invention contain less than about 1% by weight, initially and at periods up to at least 24 months. Preferably, the pharmaceutical compositions of the present invention contain less than about 0.8 % by weight, and more preferably they less than about 0.6 % by weight of N-formylDCL when such compositions were stored at about 25°C and about 60 % RH for at least 24 months.

The term "pharmaceutically acceptable basic salt" as used herein means a carbonate, phosphate, silicate or sulfate of calcium, magnesium or aluminum, or mixtures thereof. Typically suitable pharmaceutically acceptable basic salts include calcium sulfate anhydrous, hydrates of calcium sulfate, such as calcium sulfate dihydrate, magnesium sulfate anhydrous, hydrates of magnesium sulfate, dibasic calcium phosphate, dibasic calcium phosphate anhydrous, tribasic calcium phosphate, calcium silicate, magnesium silicate, magnesium trisilicate, aluminum silicate, and magnesium aluminum silicate. The use of calcium phosphate salts is preferred. The use of dibasic calcium phosphate hydrates is more preferred. The use of dibasic calcium phosphate dihydrate is most preferred.

The DCL-protective amount of a pharmaceutically acceptable basic salt is such that the pharmaceutical composition contains less than 1% by weight of N-formyldescarbonylethoxyloratadine after storage at 25°C and 60% relative humidity for at least 24 months.

The DCL-protective amount of the pharmaceutically acceptable basic salt used in the compositions of the present invention is normally about 50% by weight of the total composition. The w/w ratio of the protective amount of the pharmaceutically acceptable basic salt to the anti-allergic amount of DCL is in the range of about 5:1 to about 60:1, preferably about 7:1 to about 11:1, and most preferably about 10:1 to about 11:1.

The term "disintegrant" as used herein means a pharmaceutically acceptable material or combination of such materials that provides a pharmaceutically acceptable dissolution rate for the compositions of the present invention, preferably a dissolution rate for the compositions of the present invention of at least about 80% by weight in about 45 minutes in accordance with the USP paddle dissolution test <711> on page s 1791-1793 of USP 23/ NF 18, 1995, UNITED STATES PHARMA-COPEIAL CONVENTION, INC., Rockvile MD 20852. Normally, the dissolution rate is measured in 0.1N HCl at 37°C. The preferred dissolution rate of the compositions of the present invention is at least about 80 % by weight in about 30 minutes, and more preferably, the dissolution rate of the compositions of the present invention is at least about 90 % by weight in about 30 minutes.

Typically suitable pharmaceutically acceptable disintegrants include microcrystalline cellulose, starch, e.g., pregelatinized starch and corn starch, mannitol, croscarmellose sodium and confectioner's sugar (a mixture of at least 95% by weight sucrose and corn starch that has been ground to a fine powder).The pharmaceutical compositions of the present invention contain at least one, preferably at least two, and most preferably two pharmaceutically acceptable disintegrants in the w/w ratio of about 1:1 to 3:1. In a preferred embodiment of the present invention, the two pharmaceutically acceptable disintegrates are cellulose, and starch, preferably corn starch, in the w/w ratio of about 2:1 to about 3:1.

The w/w ratio of the protective amount of the pharmaceutically acceptable basic salt to the amount of the pharmaceutically acceptable disintegrant(s) is in the range of about 1.1:1 to about 2:1, preferably about 1.2:1 to about 1.75:1, and most preferably about 1.20:1 to about 1.25:1. The most preferred w/w ratios of the protective amount of the pharmaceutically acceptable basic salt to the amount of the phatmaceutically acceptable disintegrant(s) are 1:1 to 2:1, preferably 1.5:1 to 2:1, more preferably 1.2:1 to 1.75:1, with a ratio range of 1.25:1 to 1.75:1 being particularly preferred.

Unexpectedly, we discovered that when descarbonylethoxyloratadine was combined with a carrier medium comprising dibasic calcium phosphate, and microcrystalline cellulose-in the absence of prior art excipients such as stearic acid, or lactose - we produced a pharmaceutical composition that was stable to discoloration when stored for 4 weeks in open petri dishes at a temperature of 40°C and relative humidity of 75%. In a preferred embodiment of the present invention, the carrier medium also contains corn starch and talc. In place of the corn starch one may substitute pregelatinized starch; the talc may be replaced by PEG 8000. The calcium dibasic phosphate may be replaced by calcium sulfate dihydrate, but use of calcium dibasic phosphate is preferred. No significant changes (less than about 1-2% by weight) were observed in the physical appearance, moisture content, chemical assay of descarbonylethoxyloratadine and dissolution rate of the tablet formulations when a preferred embodiment of the present invention of Example 10 was stored in plastic bottles or blister packages for up to 9 months at 25°C/60% RH or at 30°C/60 % RH or for up to 6 months at 40°C/75 % RH.

The descarbonylethoxyloratadine used in the present invention may be prepared in accordance with Example VI of U.S. Patent No. 4,659,716. Descarbonylethoxyloratadine exists in two polymorphic forms (form 1 and form 2) which may be prepared in accordance with the Examples 1-3 and procedures of commonly-owned co-pending U.S. Patent Application Serial No. 08/886,766 filed 07/02/97. These two polymorphic forms interconverted during the manufacture of the tablets formulation of the present invention. While either polymorph form may be used, form I is preferred.

## Pharmaceutical Compositions

Pharmaceutical compositions of this invention contain an anti-allergically effective amount of descarbonylethoxyloratadine as the active ingredient, and a pharmaceutically acceptable carrier medium which may include, in addition to specific amounts of calcium dibasic phosphate and microcrystalline cellulose, other inert pharmaceutically acceptable ingredients that may be solids or liquids. Solid form compositions include powders, tablets, dispersible granules, capsules, cachets, and suppositories. The inert pharmaceutically acceptable carrier medium includes one or more substances which may also act as diluents, flavoring agents, solubilizers, lubricants, suspending agents, binders, tablet disintegration agents or encapsulating materials. The solid dosage forms of the pharmaceutical compositions of the present invention are suitable for oral administration and include powders, tablets, dispersible granules, capsules, cachets, buccals, and suppositories. In powders, the carrier medium is a finely divided solid which is in admixture with the finely divided active ingredient. In the tablet, the active ingredient is mixed with carrier medium having the necessary binding properties in suitable proportions and compacted in the shape and size desired. The anti-allergic effective amount of DCL in the pharmaceutical compositions of this invention, e.g., powders and tablets is from about 0.5 to about 15 percent, preferably about 0.5 to 10 weight percent, and more preferably about 1 to 10 weight percent. The term "compositions" is intended to include the formulation of the active compound with encapsulating material as carrier providing a capsule in which the active ingredient (with or without other carriers) is surrounded by carrier medium, which is thus in association with it. Similarly, caches are included.

The anti-allergic effective amount of descarbonylethoxyloratadine for oral administration varies from about 1 to 50 mg/day, preferably about 2.5 to 20 mg/day and more preferably about 5 to 10 mg/day in single or divided doses. The most preferred amount is 5 mg, once a day.

Of course the precise dosage and dosage regimen may be varied depending upon the requirements of the patients (e.g.. his or her sex, age) as well as the severity of the allergic condition being treated. Determination of the proper dosage and dosage regimen for a particular patient will be within the skill of the attending clinician.

Descarbonylethoxyloratadine possess antihistaminic properties. These antihistaminic properties have been demonstrated in standard animal models, such as prevention of histamine - induced lethality in guinea pigs. Antihistaminic activity of polymorph form 1 and form 2 of descarbonylethoxyloratadine has also been demonstrated in a monkey model.

General Experimental

Descarbonylethoxyloratadine may be prepared in accordance with Example VI of USP No. 4,659,716. Calcium dibasic phosphate dihydrate [$Ca(H_2PO_4)_2 \cdot 2H_2O$] is available from Rhone Poulenc Rorer, Shelton, CT 06484; microcrystalline cellulose is available from FMC Corporation Food & Pharmaceutical Products, Philadelphia, PA 19103, corn starch NF is available from National Starch & Chemical Corp., Bridgewater, NJ 08807 and the talc USP is available from Whittaker, Clark and Daniels, Inc., South Plainfield, NJ 07080.

**Method of Manufacture of Pharmaceutical Compositions of the Present Invention in the form of Tablets**

The following procedure illustrates the formulation of tablets:

Starch paste preparation

1. Prepare a 10 w/w starch paste by dispersing the paste portion of corn starch into a portion of purified water in a suitable container equipped with an agitator.
2. While mixing, heat the contents of the container to 95°C and maintain this temperature for 30 minutes.
3. Add an additional amount of purified water to the heated mixture and allow the so-formed starch paste to cool to approximately 50°C.
4. While mixing, add the descarbonylethoxyloratadine to the starch paste.

Granulation

5. To a suitable fluid bed processing bowl, charge the dibasic calcium phosphate dihydrate, a portion of the corn starch and a portion of the microcrystalline cellulose. Place the processing bowl into a fluid bed processor.
6. Fluidize the powder bed and mix for 3 minutes.
7. Begin granulating the powder by pumping the starch paste of step 4 into the fluidized bed at a suitable spray rate (for a 600,000 tablet batch size, the spray rate was 500 mL/min.) and a bed temperature of 22°C.
8. Continue to dry the granulation at 60°C until the granulation has a final loss on drying of 2% or less.
9. Pass the dried granulation through a suitable sieve or mill.
10. Charge the granulation to a suitable blender and add the requisite amount of the remaining portion of microcrystalline cellulose, corn starch, and talc. Blend for 5 minutes to produce a uniform powder blend.

The resulting blend may be filled into suitable two-piece hard gelatin capsules on a suitable encapsulating machine. The blend may also be compressed to an appropriate size and weight on a suitable tablet machine.

Tableting

1. Compress the final powder blend on a suitable tablet press with a target tablet weight of 100 mg and hardness of 7-9 s.c.u. (Strong-Cobb Units)

The tablets may be film-coated by charging the compressed tablets into suitable coating equipment having a rotating pan and heater. The tablets on the rotating pan are contacted at a temperature of about 30 - 50°C with a coating solutions formed by dissolving clear or colored coating materials in purified water. After the tablets are completely coated, a polishing powder may be added to the coated tablets to provide polished coated tablets. Alternatively, the colored coating material may be added as a dry powder in step 5 or 10, preferably step 5 of the Granulation phase of the process. It is preferred that the colored coating material is preferably substantially free, i.e., < about 1%, or more preferably completely free of offensive excipients such as lactose.

## Examples 1-5

Follow the above-listed manufacturing procedure using the ingredients listed below and compress the powder blend into tablets.

| Ingredients | 1 mg strength | 2.5 mg strength | 5 mg strength | 7.5 mg strength | 10 mg strength |
|---|---|---|---|---|---|
| | (mg/tab) | (mg/tab) | (mg/tab) | (mg/tab) | (mg/tab) |
| Descarbonylethoxyloratadine | 1 | 2.5 | 5 | 7.5 | 10 |
| Dibasic calcium phosphate dihydrate USP | 53 | 53 | 53 | 53 | 53 |
| Microcrystalline cellullose NF | 32 | 30.5 | 28 | 25.5 | 23 |
| Corn starch NF | 11 | 11 | 11 | 11 | 11 |
| Talc USP | 3 | 3 | 3 | 3 | 3 |
| Total | 100 | 100 | 100 | 100 | 100 |

## EXAMPLES 6-9

The tablet formulations of Examples 6-9 were prepared in accordance with procedure of Examples 1-5.

## Example 6

| Ingredients | mg/tablet |
|---|---|
| Descarbonylethoxyloratadine | 10 |
| Lactose monohydrate | 69 |
| Corn starch | 18 |
| Stearic acid | 2 |
| Silicon dioxide | 1 |

## Example 7

| Ingredients | mg/tablet |
|---|---|
| Descarbonylethoxyloratadine | 10 |
| Lactose monohydrate | 59 |
| Microcrystalline cellulose | 8 |
| Pregelatinized starch | 15 |

(continued)

| Ingredients | mg/tablet |
|---|---|
| Croscarmellose sodium | 5 |
| Silicon dioxide | 1 |
| Stearic acid | 2 |

**Example 8**

| Ingredients | mg/tablet |
|---|---|
| Descarbonylethoxyloratadine | 2.5 |
| Dibasic calcium phosphate Dihydrate | 78.5 |
| Corn starch | 18 |
| Magnesium stearate | 1 |

**Example 9**

| Ingredients | mg/tablet |
|---|---|
| Descarbonylethoxyloratadine | 2.5 |
| Microcrystalline cellulose | 10 |
| Mannitol | 71.5 |
| Pregelatinized starch | 15 |
| Magnesium stearate | 1 |

The tablet formulations of Examples 6-9 prepared in accordance with procedure of Examples 1-5 discolored rapidly when they were placed in open petri dishes after less than one week under a temperature of 40°C and a relative humidity of 75%.

**Color stability of formulated tablets of Examples 1-5**

The color stability of the above mentioned tablets of Examples 1-5 was studied in open petri dishes under a stressed condition of a temperature of 40°C and 75% relative humidity. After storage in the open petri dishes under this condition for 4 weeks, the tablets of Examples 1-5 were found to be free of discoloration and remained white in color. When descarbonylethoxyloratadine was formulated with other excipients such as lactose and stearic acid and former into tablets, in accordance with the procedures of Examples 1-5, the tablets of Examples 6-9 discolored rapidly after less than one week under the same storage conditions. A solid powder formulation blend(similar to that of the tablets of Examples 6) containing DCL, lactose monohydrate and stearic acid in the w/w/w/ ratio of 1:7:0.2 also decomposed rapidly after less than one week under the same storage conditions of a temperature of 40°C and 75% relative humidity; the chemical assay for descarbonylethoxyloratadine in this solid powder formulation was about 86% of the initial amount and the color of the formulation was pink.

**Example 10**

The procedures of Examples 1-5 were followed except that the formulation of Example 3 was compressed into tablets and filmed coated and polished.

| Ingredients | mg/tablet |
|---|---|
| Descarbonylethoxyloratadine | 5.0 |
| Dibasic calcium phosphate | 53.00 |

(continued)

| Ingredients | mg/tablet |
|---|---|
| Dihydrate USP Microcrystalline cellulose NF | 28.00 |
| Corn starch NF | 11.00 |
| Talc NF | 3.00 |
| Film coat(blue) | 6.00 |
| Film coat(clear) | 0.6 |
| Polishing Wax[1] | 0.01 |

1. The polishing wax is a 1:1 w/w mixture of Camuba wax and white wax.

**The stability of formulated tablets of Example 10**

Chemical assay, physical properties, and photostability of the formulated tablets of Examples 10 were measured on samples placed in high density polyethylene bottles and blister packages.

No significant changes (<1-2%) were observed in the physical appearance, moisture content, chemical assay of descarbonylethoxylpratadine and dissolution rate when the tablets of Example 10 was stored in plastic bottles or blister packages for up to 9 months at 25°C/60% relative humidity("RH") or at 30°C/60 % RH or for up to 6 months at 40°C/75 % RH. A small amount of degradation, e.g., N-formyIDCL, was observed in the tablets stored in bottles (about 0.8%) and in blisters( about 1.2%) at 40°C/75 % RH for 6 months; only about 0.2-0.3% of the degradation product was observed in any samples of the tablets stored in blisters or bottles for 9 months at 25°C/60% or at 30°C/60 % RH. It is expected that the International Conference on Harmonization("ICH") Impurity Guideline for a 5 mg tablet stored for 24 months at 25°C/60% RH or for 12 months at 30°C/60 % RH of 1% by weight of the tablet will be met. When the tablets in an open dish were subjected to ICH light conditions for one week at 25°C, the total amount of decomposition products was 0.34% by weight.

Example 11

The procedures of Examples 10 were followed except that the Blue lake was added as a dry powder to step 5 of the Granulation phase and the formulation was thereafter compressed into tablets

| Ingredients | mg/tablet |
|---|---|
| Descarbonylethoxyloratadine | 5.0 |
| Dibasic calcium phosphate Dihydrate USP | 53.00 |
| Microcrystalline cellulose NF | 27.72 |
| Corn starch NF | 11,00 |
| Talc NF | 3.00 |
| FD&C Blue #2 Lake | 0.28 |
| Total | 100.00 |

The formulation of Example 11 is expected to have similar chemical assay, and physical properties and photostability to that observed for the formulation of Example 10.

**Claims**

1. A pharmaceutical composition comprising an anti-allergic effective amount of descarbonylethoxyloratadine in a pharmaceutically acceptable carrier medium comprising a pharmaceutically acceptable basic salt, wherein the pharmaceutically acceptable carrier medium is substantially free of acidic excipients, and wherein the pharmaceutically acceptable basic salt is a carbonate, phosphate, silicate or sulfate of calcium, magnesium or aluminum or mixtures thereof.

2. A pharmaceutical composition according to Claim 1 wherein the pharmaceutically acceptable basic salt is a calcium phosphate salt.

3.  A pharmaceutical composition according to Claim 2 wherein the pharmaceutically acceptable basic salt is calcium dibasic phosphate.

4.  A pharmaceutical composition according to any preceding claim which contains less than 1% by weight of N-formyldescarbonylethoxyloratadine after storage at 25°C and 60% relative humidity for at least 24 months.

5.  A pharmaceutical composition according to any preceding claim wherein the weight/weight ("w/w") ratio of the pharmaceutically acceptable basic salt to the anti-allergic effective amount of descarbonylethoxyloratadine is in the range of 5:1 to 60:1.

6.  A pharmaceutical composition according to Claim 5 wherein the w/w ratio of the pharmaceutically acceptable basic salt to the anti-allergic effective amount of descarbonylethoxyloratadine is in the range of 7:1 to 11:1.

7.  A pharmaceutical composition according to Claim 6 wherein the w/w ratio of the pharmaceutically acceptable basic salt to the anti-allergic effective amount of descarbonylethoxyloratadine is in the range of 10:1 to 11:1.

8.  A pharmaceutical composition according to any preceding claim which is adapted for oral administration and which further comprises at least one pharmaceutically acceptable disintegrant.

9.  A pharmaceutical composition according to Claim 8 which comprises two pharmaceutically acceptable disintegrants which are microcrystalline cellulose and starch.

10. A pharmaceutical composition according to Claim 8 or Claim 9 wherein the at least one pharmaceutically acceptable disintegrant is in an amount sufficient to provide dissolution of at least 80% by weight of the pharmaceutical composition in 45 minutes.

11. A pharmaceutical composition according to any of Claims 8 to 10 wherein the w/w ratio of the pharmaceutically acceptable basic salt to said disintegrant is in the range of 1:1 to 2:1.

12. A pharmaceutical composition according to Claim 11 wherein the w/w ratio of the pharmaceutically acceptable basic salt to said disintegrant is in the range of 1.5:1 to 2:1.

13. A pharmaceutical composition according to Claim 11 wherein the w/w ratio of the pharmaceutically acceptable basic salt to said disintegrant is in the range of 1.2:1 to 1.75:1.

14. A pharmaceutical composition according to Claim 13 wherein the w/w ratio of the pharmaceutically acceptable basic salt to said disintegrant is in the range of 1.25:1 to 1.75:1.

15. A pharmaceutical composition according to Claim 3 which comprises:

| Ingredient | Amount (weight %) |
| --- | --- |
| Descarbonylethoxyloratadine | 0.5 - 15 |
| Calcium Dibasic Phosphate Dihydrate USP | 10 - 90 |
| Microcrystalline Cellulose NF | 5 - 60 |
| Corn starch NF | 1 - 60 |
| Talc USP | 0.5 - 20. |

16. A pharmaceutical composition according to Claim 3 which comprises:

| Ingredient | Amount (weight %) |
| --- | --- |
| Descarbonylethoxyloratadine | 0.5 - 15 |
| Calcium Dibasic Phosphate Dihydrate USP | 45 - 60 |
| Microcrystalline Cellulose NF | 20 - 40 |

(continued)

| Ingredient | Amount (weight %) |
|---|---|
| Corn starch NF | 5 - 15 |
| Talc USP | 1 - 10. |

**17.** A pharmaceutical composition according to Claim 3 which comprises:

| Ingredient | Amount (weight %) |
|---|---|
| Descarbonylethoxyloratadine | 1 - 10 |
| Calcium Dibasic Phosphate Dihydrate USP | 50 - 56 |
| Microcrystalline Cellulose NF | 25 - 35 |
| Corn starch NF | 10 - 12 |
| Talc USP | 2 - 5. |

**18.** A pharmaceutical composition according to any preceding claim wherein the amount of descarbonylethoxyloratadine is in the range of 1 to 10 weight percent.

**19.** A pharmaceutical composition of any preceding claim which initially contains less than 1 % by weight of N-formyldescarbonylethoxyloratadine.

**Patentansprüche**

**1.** Pharmazeutische Zusammensetzung, die eine antiallergisch wirksame Menge Descarbonylethoxyloratadin in einem pharmazeutisch annehmbaren Trägermedium enthält, das ein pharmazeutisch annehmbares basisches Salz enthält, wobei das pharmazeutisch annehmbare Trägermedium im Wesentlichen frei von sauren Hilfsstoffen ist, und wobei das pharmazeutisch annehmbare basische Salz ein Carbonat, Phosphat, Silikat oder Sulfat von Calcium, Magnesium oder Aluminium oder Mischungen davon ist.

**2.** Pharmazeutische Zusammensetzung nach Anspruch 1, bei der das pharmazeutisch annehmbare basische Salz ein Calciumphosphatsalz ist.

**3.** Pharmazeutische Zusammensetzung nach Anspruch 2, bei der das pharmazeutisch annehmbare basische Salz Dicalciumorthophosphat ist.

**4.** Pharmazeutische Zusammensetzung nach einem der vorhergehenden Ansprüche, die nach Lagerung bei 25°C und 60 % relativer Feuchtigkeit für mindestens 24 Monate weniger als 1 Gew.% N-Formyldescarbonylethoxyloratadin enthält.

**5.** Pharmazeutische Zusammensetzung nach einem der vorhergehenden Ansprüche, bei der das Gewicht/Gewicht-("w/w")-Verhältnis des pharmazeutisch annehmbaren basischen Salzes zu der antiallergisch wirksamen Menge des Descarbonylethoxyloratadins im Bereich von 5:1 bis 60:1 ist.

**6.** Pharmazeutische Zusammensetzung nach Anspruch 5, bei der das w/w-Verhältnis des pharmazeutisch annehmbaren basischen Salzes zu der antiallergisch wirksamen Menge des Descarbonylethoxyloratadins im Bereich von 7:1 bis 11:1 ist.

**7.** Pharmazeutische Zusammensetzung nach Anspruch 6, bei der das w/w-Verhältnis des pharmazeutisch annehmbaren basischen Salzes zu der antiallergisch wirksamen Menge des Descarbonylethoxyloratadins im Bereich von 10:1 bis 11:1 ist.

**8.** Pharmazeutische Zusammensetzung nach einem der vorhergehenden Ansprüche, die zur oralen Verabreichung angepasst ist und ferner mindestens ein pharmazeutisch annehmbares Sprengmittel enthält.

**9.** Pharmazeutische Zusammensetzung nach Anspruch 8, die zwei pharmazeutisch annehmbare Sprengmittel enthält, die mikrokristalline Cellulose und Stärke sind.

**10.** Pharmazeutische Zusammensetzung nach Anspruch 8 oder 9, bei der das mindestens eine pharmazeutisch annehmbare Sprengmittel in einer Menge vorliegt, die ausreicht, um Auflösung von mindestens 80 Gew.% der pharmazeutischen Zusammensetzung in 45 Minuten zu bewirken.

**11.** Pharmazeutische Zusammensetzung nach einem der vorhergehenden Ansprüche, bei der das w/w-Verhältnis des pharmazeutisch annehmbaren basischen Salzes zu dem Sprengmittel im Bereich von 1:1 bis 2:1 ist.

**12.** Pharmazeutische Zusammensetzung nach Anspruch 11, bei der das w/w-Verhältnis des pharmazeutisch annehmbaren basischen Salzes zu dem Sprengmittel im Bereich von 1,5:1 bis 2:1 ist.

**13.** Pharmazeutische Zusammensetzung nach Anspruch 11, bei der das w/w-Verhältnis des pharmazeutisch annehmbaren basischen Salzes zu dem Sprengmittel im Bereich von 1,2:1 bis 1,75:1 ist.

**14.** Pharmazeutische Zusammensetzung nach Anspruch 13, bei der das w/w-Verhältnis des pharmazeutisch annehmbaren basischen Salzes zu dem Sprengmittel im Bereich von 1,25:1 bis 1,75:1 ist.

**15.** Pharmazeutische Zusammensetzung nach Anspruch 3, die

| Bestandteil | Menge (Gew.%) |
|---|---|
| Descarbonylethoxyloratadin | 0,5 bis 15 |
| Dicalciumorthophosphat-Dihydrat USP | 10 bis 90 |
| mikrokristalline Cellulose NF | 5 bis 60 |
| Maisstärke NF | 1 bis 60 |
| Talkum USP | 0,5 bis 20 |

enthält.

**16.** Pharmazeutische Zusammensetzung nach Anspruch 3, die

| Bestandteil | Menge (Gew.%) |
|---|---|
| Descarbonylethoxyloratadin | 0,5 bis 15 |
| Dicalciumorthophosphat-Dihydrat USP | 45 bis 60 |
| mikrokristalline Cellulose NF | 20 bis 40 |
| Maisstärke NF | 5 bis 15 |
| Talkum USP | 1 bis 10 |

enthält.

**17.** Pharmazeutische Zusammensetzung nach Anspruch 3, die

| Bestandteil | Menge (Gew.%) |
|---|---|
| Descarbonylethoxyloratadin | 1 bis 10 |
| Dicalciumorthophosphat-Dihydrat USP | 50 bis 56 |
| mikrokristalline Cellulose NF | 25 bis 35 |
| Maisstärke NF | 10 bis 12 |
| Talkum USP | 2 bis 5 |

enthält.

**18.** Pharmazeutische Zusammensetzung nach einem der vorhergehenden Ansprüche, bei der die Menge an Descarbonylethoxyloratadin im Bereich von 1 bis 10 Gew.% liegt.

**19.** Pharmazeutische Zusammensetzung nach einem der vorhergehenden Ansprüche, die anfangs weniger als 1 Gew. % N-Formyldescarbonylethoxyloratadin enthält.

**Revendications**

**1.** Composition pharmaceutique, comprenant de la décarbonyl-éthoxyloratadine en une quantité à effet anti-allergique, dans un milieu véhicule pharmacologiquement admissible qui comprend un sel basique pharmacologiquement admissible, dans laquelle composition le milieu véhicule pharmacologiquement admissible ne contient pratiquement pas d'excipients acides et le sel basique pharmacologiquement admissible est un carbonate, un phosphate, un silicate ou un sulfate de calcium, de magnésium ou d'aluminium, ou un mélange de tels sels.

**2.** Composition pharmaceutique conforme à la revendication 1, dans laquelle le sel basique pharmacologiquement admissible est un phosphate de calcium.

**3.** Composition pharmaceutique conforme à la revendication 2, dans laquelle le sel basique pharmacologiquement admissible est de l'hydrogénophosphate de calcium.

**4.** Composition pharmaceutique conforme à l'une des revendications précédentes, qui contient moins de 1 % en poids de N-formyl-décarbonyléthoxyloratadine au bout d'au moins 24 mois de stockage à 60 % d'humidité relative et à 25 °C.

**5.** Composition pharmaceutique conforme à l'une des revendications précédentes, dans laquelle le rapport pondéral (p/p) du sel basique pharmacologiquement admissible à la quantité à effet anti-allergique de décarbonyl-éthoxyloratadine vaut de 5/1 à 60/1.

**6.** Composition pharmaceutique conforme à la revendication 5, dans laquelle le rapport pondéral (p/p) du sel basique pharmacologiquement admissible à la quantité à effet anti-allergique de décarbonyl-éthoxyloratadine vaut de 7/1 à 11/1.

**7.** Composition pharmaceutique conforme à la revendication 6, dans laquelle le rapport pondéral (p/p) du sel basique pharmacologiquement admissible à la quantité à effet anti-allergique de décarbonyl-éthoxyloratadine vaut de 10/1 à 11/1.

**8.** Composition pharmaceutique conforme à l'une des revendications précédentes, qui est adaptée en vue d'une administration par voie orale et qui contient en outre au moins un désintégrant pharmacologiquement admissible.

**9.** Composition pharmaceutique conforme à la revendication 8, qui contient deux désintégrants pharmacologiquement admissibles qui sont une cellulose microcristalline et un amidon.

**10.** Composition pharmaceutique conforme à la revendication 8 ou 9, dans laquelle le désintégrant pharmacologiquement admissible au nombre d'au moins un se trouve en une quantité suffisante pour assurer la dissolution, en 45 minutes, d'au moins 80 % en poids de la composition pharmaceutique.

**11.** Composition pharmaceutique conforme à l'une des revendications 8 à 10, dans laquelle le rapport pondéral du sel basique pharmacologiquement admissible audit désintégrant vaut de 1/1 à 2/1.

**12.** Composition pharmaceutique conforme à la revendication 11, dans laquelle le rapport pondéral du sel basique pharmacologiquement admissible audit désintégrant vaut de 1,5/1 à 2/1.

**13.** Composition pharmaceutique conforme à la revendication 11, dans laquelle le rapport pondéral du sel basique pharmacologiquement admissible audit désintégrant vaut de 1,2/1 à 1,75/1.

**14.** Composition pharmaceutique conforme à la revendication 13, dans laquelle le rapport pondéral du sel basique pharmacologiquement admissible audit désintégrant vaut de 1,25/1 à 1,75/1.

**15.** Composition pharmaceutique conforme à la revendication 3, qui comprend :

| Ingrédient | Quantité (% en poids) |
|---|---|
| Décarbonyl-éthoxyloratadine | 0,5 - 15 |
| Hydrogénophosphate de calcium dihydraté (USP) | 10 - 90 |
| Cellulose microcristalline NF | 5 - 60 |
| Amidon de maïs NF | 1 - 60 |
| Talc USP | 0,5 - 20 |

**16.** Composition pharmaceutique conforme à la revendication 3, qui comprend :

| Ingrédient | Quantité (% en poids) |
|---|---|
| Décarbonyl-éthoxyloratadine | 0,5 - 15 |
| Hydrogénophosphate de calcium dihydraté (USP) | 45 - 60 |
| Cellulose microcristalline NF | 20 - 40 |
| Amidon de maïs NF | 5 - 15 |
| Talc USP | 1 - 10 |

**17.** Composition pharmaceutique conforme à la revendication 3, qui comprend :

| Ingrédient | Quantité (% en poids) |
|---|---|
| Décarbonyl-éthoxyloratadine | 1 - 10 |
| Hydrogénophosphate de calcium dihydraté (USP) | 50 - 56 |
| Cellulose microcristalline NF | 25 - 35 |
| Amidon de maïs NF | 10 - 12 |
| Talc USP | 2 - 5 |

**18.** Composition pharmaceutique conforme à l'une des revendications précédentes, dans laquelle la proportion pondérale de décarbonyl-éthoxyloratadine vaut de 1 à 10 %.

**19.** Composition pharmaceutique conforme à l'une des revendications précédentes, qui contient initialement moins de 1 % en poids de N-formyl-décarbonyl-éthoxyloratadine.